Europäisches Patentamt

(19) European Patent Office

Office européen des brevets

(11) Numéro de publication: **0 079 822**
**B1**

(12) **FASCICULE DE BREVET EUROPEEN**

(45) Date de publication du fascicule du brevet:
27.12.84

(21) Numéro de dépôt: **82402033.3**

(22) Date de dépôt: **05.11.82**

(51) Int. Cl.³: **B 01 J 7/02, C 12 M 1/04**

(54) **Dispositif permettant d'obtenir une atmosphère enrichie en gaz carbonique et/ou exempte d'oxygène, dans un récipient étanche.**

(30) Priorité: **13.11.81 FR 8121285**

(43) Date de publication de la demande:
**25.05.83 Bulletin 83/21**

(45) Mention de la délivrance du brevet:
**27.12.84 Bulletin 84/52**

(84) Etats contractants désignés:
**AT BE CH DE GB IT LI LU NL SE**

(56) Documents cités:
**EP - A - 0 017 314**
**EP - A - 0 039 753**
**DE - A - 2 800 437**
**US - A - 4 012 203**
**US - A - 4 013 422**
**US - A - 4 347 222**

(73) Titulaire: **BIOMERIEUX, Société Anonyme dite, Marcy l'Etoile, F-69260 Charbonnières-les-Bains (FR)**

(72) Inventeur: **Trouyez, Gérard, 30, rue Chaziere, F-69004 Lyon (FR)**

(74) Mandataire: **Gillard, Marie-Louise et al, Cabinet Beau de Loménie 55, Rue d'Amsterdam, F-75008 Paris (FR)**

## Description

L'invention concerne un dispositif générateur de gaz, permettant d'obtenir, dans un récipient étanche, une atmosphère enrichie en gaz carbonique et/ou exempte d'oxygène, particulièrement favorable à la culture de micro-organismes.

On connaît déjà des dispositifs du même type contenant un ou plusieurs comprimés capables de réagir avec un liquide au contact duquel on les met au moment de l'utilisation du dispositif, en dégageant du gaz carbonique et/ou de l'hydrogène.

Ainsi, le brevet américain No 4013422 décrit un dispositif constitué par un tube souple ouvert à une extrémité, dans lequel se trouvent une ampoule contenant un liquide, un ou plusieurs comprimés d'un ou de plusieurs produits capables de réagir avec le liquide pour former du $CO_2$ et/ou de l'hydrogène et, en aval du ou des comprimés, un tampon absorbant pouvant retenir le liquide, mais laissant passer les gaz formés vers l'extrémité ouverte du tube. Le tube souple peut comporter également, à son extrémité ouverte, un dessiccateur et, dans le cas d'un dégagement d'hydrogène, un catalyseur de réaction de l'hydrogène avec l'oxygène ambiant qui est ainsi éliminé.

Le tube souple est placé dans une enceinte étanche, par exemple un emballage en matière plastique, à côté du tube à gélose ou de la boîte de Petri contenant le milieu de culture et, l'enceinte étant hermétiquement close, on libère le liquide au moment de l'emploi en brisant l'ampoule par écrasement du tube souple entre les doigts.

Le dispositif selon l'invention est constitué de façon caractéristique par une coque thermoformée soudée sur un support plan dont la face extérieure est munie d'une bande adhésive et il comprend, entre la coque et le support, dans des alvéoles communiquant avec l'extérieur par des canaux, un ou plusieurs comprimés de produits générateurs de gaz, posés sur un tampon absorbant dont au moins une face latérale est ouverte à l'extérieur et qui est susceptible de retenir, lors de l'immersion du dispositif, la quantité de liquide nécessaire à la réaction des produits générateurs de gaz.

L'un des avantages du dispositif selon l'invention par rapport aux dispositifs connus est qu'on peut l'utiliser directement dans une boîte de Petri étanche contenant le milieu de culture, la bande adhésive du support permettant de coller le dispositif sur le couvercle de cette boîte. Il n'est donc pas nécessaire de recourir à un emballage supplémentaire contenant le dispositif à côté de la boîte de Petri.

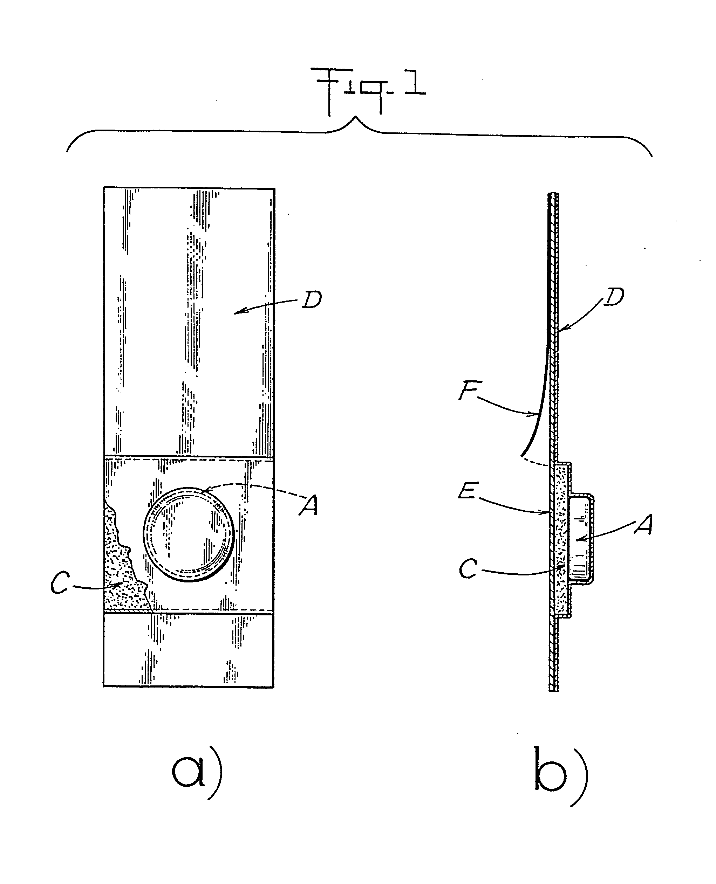

Les fig. 1, 2 et 3 ci-après représentent diverses réalisations du dispositif selon l'invention et comportent une vue de dessus a et une vue de profil b.

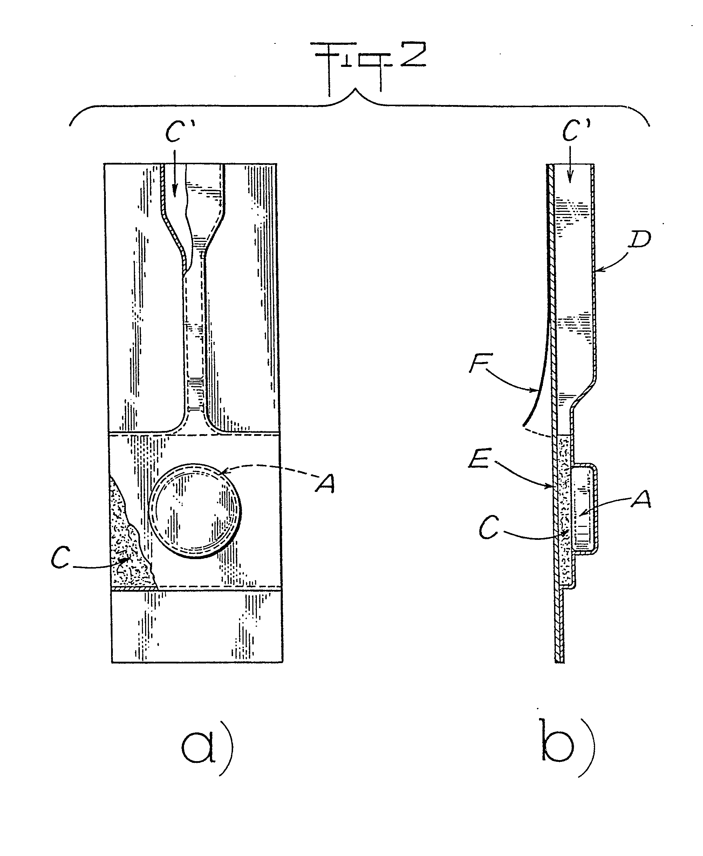

Les fig. 1 et 2 représentent un dispositif permettant d'obtenir une atmosphère enrichie en $CO_2$.

Un comprimé A d'un produit capable de dégager du gaz carbonique est posé sur un coussinet C d'une substance absorbante de type feutre, non-tissé, papier-filtre, gaze et autre, susceptible de retenir, après immersion, la quantité de liquide nécessaire à la réaction du produit dégageant le $CO_2$.

Le coussinet C et le comprimé A sont emprisonnés entre la coque thermoformée D et le support E constitué par une feuille d'une matière imperméable aux gaz et thermosoudable, telle qu'un complexe aluminium.

La coque D, qui est en une matière thermoplastique, est soudée sur le support E, laissant ouvertes à l'extérieur les faces latérales du coussinet C.

Dans la forme de réalisation de la fig. 2, la coque thermoformée D comporte, en plus de l'alvéole dans laquelle est logée le comprimé A, un canal C' ménagé entre le support et la coque et qui, d'un côté, aboutit au comprimé A et, de l'autre côté, est ouvert à l'extérieur.

Avant d'utiliser le dispositif, on imbibe le coussinet C de la quantité de liquide nécessaire à la réaction avec le produit A, soit en immergeant quelques secondes le dispositif de la fig. 1 dans le liquide, soit en déposant quelques gouttes de ce liquide, à l'aide d'un compte-gouttes par exemple, dans le canal C' du dispositif de la fig. 2.

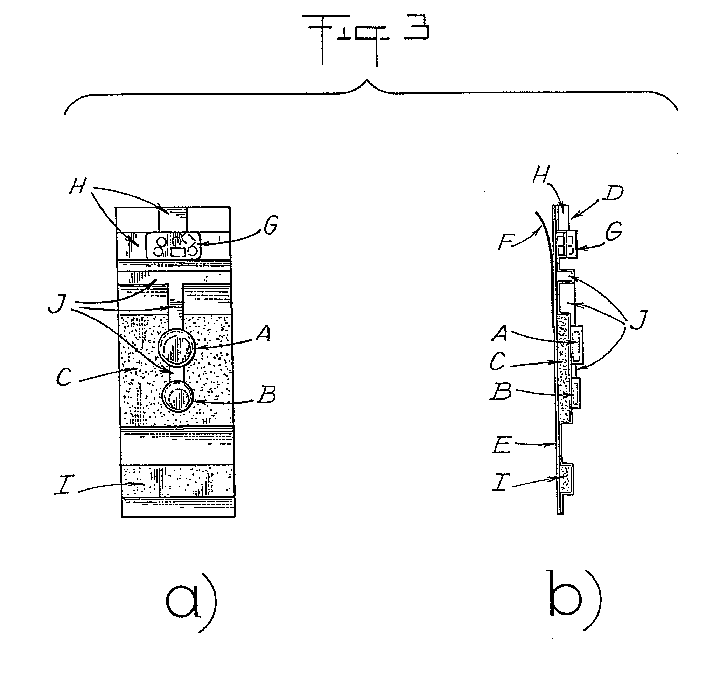

Le dispositif représenté dans la fig. 3 permet d'obtenir une atmosphère enrichie en $CO_2$ et exempte d'oxygène.

La coque thermoformée D comporte les diverses alvéoles suivantes:
— l'alvéole A reçoit le comprimé de produit dégageant du gaz carbonique;
— l'alvéole B reçoit le comprimé de produit dégageant de l'hydrogène;
— l'alvéole G reçoit un catalyseur à base de palladium (ou tout autre catalyseur d'oxydation de l'hydrogène); elle communique avec l'extérieur par les canaux H; l'hydrogène formé et l'oxygène ambiant, passant sur ce catalyseur, réagissent et l'oxygène est ainsi éliminé;
— un logement C reçoit le coussinet de substance absorbante sur lequel reposent les comprimés A et B, cette substance étant susceptible de retenir, après immersion, la quantité de liquide nécessaire à la réaction des produits générateurs de $CO_2$ et d'hydrogène;
— un logement I reçoit un tampon absorbant de type feutre, non-tissé, papier-filtre, gaze, etc., imprégné d'un produit indicateur de la présence d'oxygène; les logements C et I sont tels que les tampons absorbants qu'ils recouvrent communiquent avec l'extérieur par leurs faces latérales;
— le dégagement des gaz formés par les comprimés A et B se fait par les canaux J.

La coque D est operculée par un support E, en une matière imperméable aux gaz et thermosoudable, et comportant, sur sa face extérieure, une bande adhésive F permettant de fixer le dispositif à l'intérieur du récipient étanche et en particulier sur le couvercle de la boîte de Petri.

Au moment de l'utilisation, le dispositif est immergé quelques secondes dans le liquide (en général de l'eau) capable de réagir avec les produits générateurs de $CO_2$ et d'hydrogène. Puis on fixe le dispositif, grâce à la bande adhésive, dans un récipient étanche de volume adéquat, et en particulier sur le couvercle à l'intérieur d'une boîte de Petri étanche, contenant le milieu de culture.

La boîte de Petri étanche peut être du type décrit dans la demande de brevet français N° 80.16041. L'anaérobiose se développe dans le récipient étanche en un temps relativement court, de l'ordre de 30 min. Les parois du récipient étanche étant transparentes, on peut observer les cultures individuellement sans ouverture du récipient, permettant ainsi de suivre journellement la croissance des germes.

Pour sa conservation avant l'utilisation, le dispositif selon l'invention est emballé unitairement en un sachet parfaitement étanche aux gaz et à l'humidité; il peut être également conditionné par plusieurs unités dans un flacon étanche muni d'un déshydratant.

La simplicité du dispositif selon l'invention permet de le fabriquer en continu à partir d'une machine de thermoformage d'une matière plastique. Il peut être fixé directement par collage automatique contre le couvercle des boîtes de Petri étanches, et commercialisé avec les milieux de culture prêts en boîte, ce qui évite le suremballage.

**Revendications**

1. Dispositif générateur de gaz, permettant d'obtenir une atmosphère enrichie en gaz carbonique et/ou exempte d'oxygène dans un récipient étanche, et contenant un ou plusieurs comprimés d'un ou de plusieurs produits capables de dégager du gaz carbonique et/ou de l'hydrogène par réaction avec un liquide au contact duquel on le ou les met au moment de l'utilisation du dispositif, caractérisé en ce qu'il est constitué par une coque thermoformée (D) soudée sur un support plan (E) dont la face extérieure est munie d'une bande adhésive (F) et qu'il comprend, entre la coque et le support, dans des alvéoles (A et B) communiquant avec l'extérieur par des canaux (J et H) un ou plusieurs comprimés de produits générateurs de gaz, posés sur un tampon absorbant (C) dont au moins une face latérale est ouverte à l'extérieur et qui est susceptible de retenir, après immersion du dispositif, la quantité de liquide nécessaire à la réaction des produits générateurs de gaz.

2. Dispositif selon la revendication 1, caractérisé en ce qu'il comprend également, entre la coque et le support, dans une alvéole (G) communiquant avec l'extérieur par des canaux, un catalyseur de réaction de l'hydrogène dégagé par l'un des comprimés avec l'oxygène ambiant.

3. Dispositif selon l'une des revendications 1 ou 2, caractérisé en ce qu'il comprend, dans une alvéole entre la coque et le support, un tampon absorbant (I) imprégné d'un produit indicateur de la présence d'oxygène, et dont au moins une face latérale est ouverte à l'extérieur.

4. Procédé d'utilisation du dispositif selon l'une des revendications 1 à 3, pour créer une atmosphère enrichie en CO₂ et/ou exempte d'oxygène dans un récipient étanche, caractérisé en ce qu'on immerge le dispositif pendant quelques secondes dans le liquide capable de réagir avec le ou les produits générateurs de gaz et on colle le dispositif sur les parois du récipient que l'on ferme, immédiatement après, de façon étanche.

5. Procédé selon la revendication 4, caractérisé en ce que le dispositif est collé, après immersion, sur la paroi intérieure du couvercle d'une boîte de Petri étanche contenant un milieu de culture de micro-organismes.

**Patentansprüche**

1. Gasgeneratorvorrichtung zur Erzielung einer an Kohlensäuregas angereicherten und/oder sauerstofffreien Atmosphäre in einem dichten Behälter, welche einen oder mehrere Presskörper eines oder mehrerer zur Freisetzung von Kohlensäuregas und/oder Wasserstoff durch Reaktion mit einer Flüssigkeit, mit der man ihn (sie) bei Verwendung der Vorrichtung in Kontakt bringt, fähiger Produkte enthält, dadurch gekennzeichnet, dass sie durch eine wärmegeformte Schale (D) gebildet ist, die auf einen ebenen Träger (E), dessen Aussenfläche mit einem Klebeband (F) versehen ist, geschweisst ist, und dass sie zwischen der Schale und dem Träger in durch Kanäle (J und H) mit aussen kommunizierenden Zellen (A und B) einen oder mehrere Presskörper aus gaserzeugenden Produkten umfasst, die auf einem absorbierenden Kissen (C) angeordnet sind, von dem zumindest eine Seitenfläche nach aussen offen ist und das nach Eintauchen der Vorrichtung die für die Reaktion der gaserzeugenden Produkte notwendige Flüssigkeitsmenge zurückhalten kann.

2. Vorrichtung nach Anspruch 1, dadurch gekennzeichnet, dass sie weiters zwischen der Schale und dem Träger in einer durch Kanäle mit aussen kommunizierenden Zelle (G) einen Katalysator für die Reaktion des von einem der Presskörper freigesetzten Wassestoffs mit dem Umgebungssauerstoff umfasst.

3. Vorrichtung nach einem der Ansprüche 1 oder 2, dadurch gekennzeichnet, dass sie in einer Zelle zwischen der Schale und dem Träger ein absorbierendes Kissen (I) umfasst, das mit einem das Vorhandensein von Sauerstoff anzeigenden Produkt imprägniert ist und von dem zumindest eine Seitenfläche nach aussen offen ist.

4. Verfahren zur Verwendung einer Vorrichtung nach einem der Ansprüche 1 bis 3 zur Schaffung einer an CO₂ angereicherten und/oder von Sauerstoff freien Atmosphäre in einem dichten Gefäss, dadurch gekennzeichnet, dass man die Vorrichtung einige Sekunden lang in die mit dem (oder den) gaserzeugenden Produkt(en) reaktionsfähige Flüssigkeit taucht und die Vorrichtung auf die Wände des Gefässes klebt, das man unmittelbar danach auf dichte Weise verschliesst.

5. Verfahren nach Anspruch 4, dadurch gekennzeichnet, dass die Vorrichtung nach Eintauchen auf die Innenwand des Deckels einer dichten, ein Kulturmedium für Mikroorganismen enthaltenden Petrischale geklebt wird.

## Claims

1. Gas-generating device, permitting to obtain a carbon dioxide-enriched and/or oxygen-free atmosphere inside a tight container, and containing one or more tablets of one or more products capable of generating carbon dioxide and/or hydrogen by reaction with a liquid in the contact of which it is (or they are) placed when the device is being used, characterized in that said device is constituted by a thermoshaped shell (D), welded on a flat support (E), the external face of which is provided with an adhesive strip (F), and it contains, between the shell and the support, in compartiments (A and B) which communicate with the outside *via* conduits (J and H), one or more tablets of gas-generating substances, resting on an absorbent pad (C) at least one side face of which is exposed to the outside, and which, when the device is immersed, is capable to hold back the quantity of liquid necessary for reacting of the gas-generating products.

2. Device according to Claim 1, characterized in that said device further comprises, between the shell and the support, inside a compartment (G) communicating with the outside *via* conduits, a catalyst for the reaction between the hydrogen released by one of said tablets and the ambient oxygen.

3. Device according to one of Claims 1 or 2, characterized in that said device comprises inside a compartment situated between the shell and the support, an absorbent pad (I) impregnated with the product indicating the presence of oxygen, at least one side face of which pad is exposed to the outside.

4. Method for using the device according to one of Claims 1 to 3, for generating a carbon dioxide-enriched and/or oxygen-free atmosphere inside a tight container, characterized in that the device is immersed for a few seconds in the liquid capable of reacting with the gaz-generating product(s), and said device is adhesively fixed on the walls of the container, which latter is tightly closed immediately after.

5. Method according to Claim 4, characterized in that the device is adhesively fixed, after immersion, on the inner wall of the lid of a tight Petri dish containing a microorganisms culture medium.

# Fig.1

a)                    b)

Fig 2

a)    b)

Fig. 3

a)

b)